# EUROPÄISCHE PATENTANMELDUNG

(11) **EP 1 609 787 A1**
(43) Veröffentlichungstag der Anmeldung: **28.12.2005**
(21) Anmeldenummer: 04102730.1
(22) Anmeldetag: 15.06.2004
(51) Int. Cl.: C07D 319/06

(54) **Verfahren zur Herstellung von 4-(alpha-Hydroxyalkyl)-1,3-dioxan-5-onen**

(71) Anmelder: Girindus AG, 51402 Bensberg (DE)
(72) Erfinder: Westermann, Bernhard., 33102 Paderborn (DE); Lange, Meinolf., 72181 Starzach-Felldorf (DE); Hayat, Nasir., 33647 Bielefeld (DE); Neuhaus, Christiane., 06112 Halle (DE); Link, Fritz., 51427 Bensberg (DE)
(74) Vertreter: von Kreisler, Alek

(57) **Zusammenfassung**

Verfahren zur Synthese von Verbindungen der allgemeinen Formel 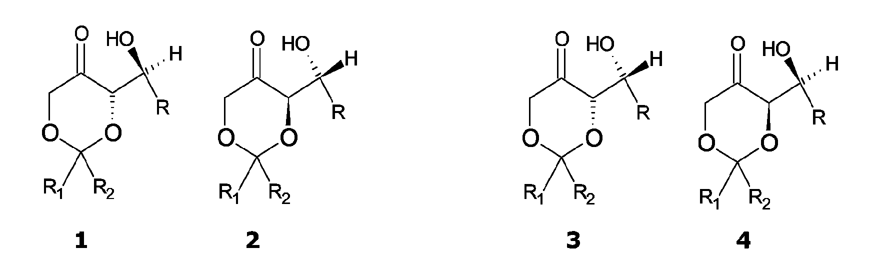 umfassend den Schritt der Umsetzung von 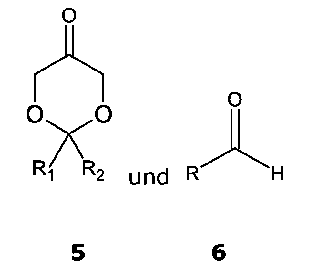 in Gegenwart von 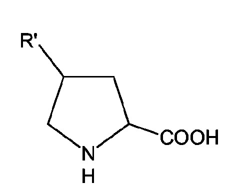 wobei
R = substituierte oder unsubstituierte Alkyl, Aryl, Heterozyklen enthaltend ein oder mehr O, N, S, P oder B;
R₁ und R₂ unabhängig voneinander H, substituierte oder unsubstituierte Alkyl, Aryl, Heterozyklen enthaltend ein oder mehr O, N, S, P oder B
R' = H, OH oder SiX₃ wobei X unabhängig voneinander Alkyl oder Aryl sind.

## Beschreibung

Die vorliegende Erfindung betrifft ein Syntheseverfahren und dadurch erhältliche Verbindungen.

Aldolreaktionen sind eine der wesentlichen Kohlenstoff/Kohlenstoff verknüpfende Reaktionen sowohl in der Natur als auch im Repertoire des synthetischen Chemikers. In der Natur werden solche Reaktionen katalysiert von Enzymen, die entweder über einen Enamin-Mechanismus (Klasse 1 Aldolasen) oder über einen Zink-Kofaktor (Klasse 2 Aldolasen) funktionieren. Hierbei wird eine hohe Stereoselektivität erreicht.

Trotz der bahnbrechenden Entwicklungen der stereoselektiven, metallkatalysierten Aldolreaktion in den vergangenen Jahren (siehe hierzu: "Comprehensive Asymmetric Catalysis"; Kapitel 29, ed. Eric N. Jacobsen, Andreas Pflatz, Hisahi Yamamoto, Springer Verlag, Berlin, 2002) konnten Erfolge mittels organokatalytischer Methoden mit einer großen Substratbreite erst in jüngerer Zeit vorgestellt werden.

All diese Methoden ergeben aber schlechte Ausbeuten und Selektivitäten, wenn das natürliche, phosphorylierte Substrat der Aldolasen, Dihydroxyaceton (DHA), eingesetzt wird.

Es sind entsprechende Aldolreaktionen mit zyklischen sekundären Aminen in organischen Lösungsmitteln und in wässrigen Medien bekannt; vgl. A. Córdova, W. Notz and C.F. Barbas III. in Chem. Commun. 202, Seiten 3024 bis 3025. Die dort beobachteten Stereoselektivitäten sind überwiegend gering.

Aufgabe der vorliegenden Erfindung war es, ein verbessertes Syntheseverfahren mit hoher Stereoselektivität auf Grundlage von Aldolreaktionen zu entwickeln.

Gelöst wird die Aufgabe durch ein Verfahren zur Synthese von Verbindungen der allgemeinen Formel 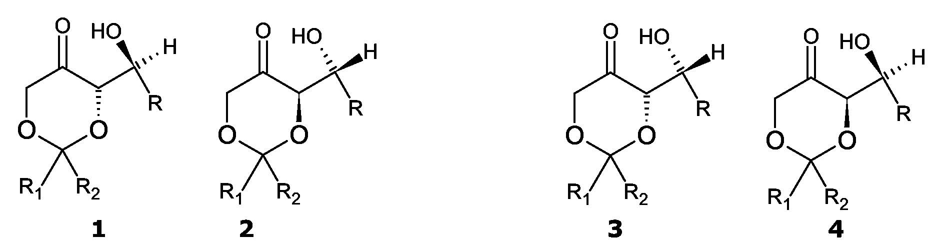 umfassend den Schritt der Umsetzung von 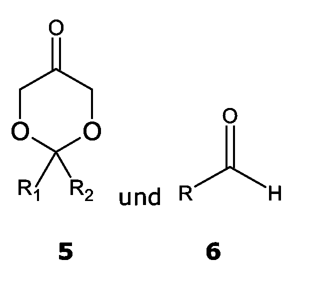 in Gegenwart von 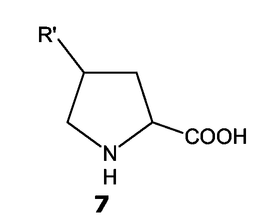 wobei
R = substituierte oder unsubstituierte Alkyl, Aryl, Heterozyklen enthaltend ein oder mehr O, N, S, P oder B;
R₁ und R₂ unabhängig voneinander H, substituierte oder unsubstituierte Alkyl, Aryl, Heterozyklen enthaltend ein oder mehr O, N, S, P oder B
R' = H, OH oder SiX₃ wobei X unabhängig voneinander Alkyl oder Aryl sind.

Erfindungsgemäß wird mit einer zyklischen Ausgangsverbindung, nämlich Derivaten des 1,3-Dioxan-5-on gearbeitet, die in Gegenwart von Prolin oder Prolinderivaten mit Aldehyden umgesetzt werden. Es werden hohe Stereoselektivitäten erreicht.

"Alkyl" im Sinne dieser Anmeldung sind insbesondere gradkettige oder verzweigte Alkyl-, Cycloalkyl-, Bicycloalkyl-, Tricycloalkyl-, Alkenyl-, Alkinyl-, Heterocycloalkyl-Verbindungen. Typische Vertreter sind Methyl-, Ethyl-, N-Propyl-, Isopropyl-, Butyl-, Cyclohexyl- oder substituierte Derivate hiervon.

"Aryl" im Sinne dieser Anmeldung umfasst insbesondere auch Heteroaryl-, Arylalkyl- oder Heteroarylalkyl-Gruppen. Typische Vertreter sind Phenyl-, Benzyl-, Pyrridin-, sowie substituierte Derivate hiervon.

Dabei können die Alkyl- oder Aryl-Reste auch durch ein oder mehrere Hydroxy-, Alkoxy, Aryloxy-, Alkanoyl-, Aroyl-, Carboxy-, Alkoxycarbonyl-, Amino-, Alkylamino-, Hydroxylamino-, Amido-, Carbamoyl-, Ureido-, Amidino-, Guanidino-, Cyano-, Azido-, Mercapto-, Alkylthio-, Alkylsulfoxy-, Alkylsulfonyl-, Alkylsulfenyl-, Aminosulfonyl-, Fluor-, Chlor-, Brom-, Jod-, Alkyl- oder Perfluoralkyl-Reste substituierte Derivate sein.

In einer bevorzugten Ausführungsform enthält R ein oder mehr Stickstoffatome. Es handelt sich dann um Derivate von Verbindungen mit beispielsweise Azetidin-, Pyrrolidin-, Pyrrolin-, Piperidin-, Piperazin-, Homopiperazin-, Morpholin-, Thiomorpholin-, Pyridin-, Di- oder Tetra-hydropyridin-, Pyrimidin-, Pyrazin-, Azepin-, Dihydroazepin-, Oxazepin-, Diazepin-, Imidazol-, Pyrazol-, Oxazol- oder Thiazol-Ringen, die gegebenenfalls ankondensierte aliphatische, heteroaliphatische, aromatische oder heteroaromatische Ringe aufweisen und/oder durch einen oder mehrere Hydroxy-, Alkoxy-, Aryloxy-, Aklanoyl-, Aroyl-, Carboxy-, Alkoxycarbonyl-, Amino-, Alkylamino-, Hydroxylamino-, Amido-, Carbanoyl-, Ureido-, Amidino-, Guanidino-, Cyano-, Azido-, Mercapto-, Alkylthio-, Alkylsulfoxy-, Alkylsulfonyl-, Alkylsulfenyl-, Aminosulfonyl-, Fluor-, Chrom-, Brom-, Jod-, Alkyl- oder Perfluoralkyl-Reste substituiert sind.

Die zur Umsetzung notwendigen Mengen an Prolin bzw. Prolinderivat betragen typischerweise 0,1 bis 30 Mol%, mehr bevorzugt 1 bis 10 Mol%, noch mehr bevorzugt 1 bis 5 Mol%.

Um Verbindungen der Strukturformel **1, 3** zu erhalten, erfolgt die Umsetzung mit Hilfe von L-Prolin. Um die Verbindungen **2, 4** zu erhalten, wird typischerweise D-Prolin eingesetzt. Alternativ kann auch 4-Hydroxyprolin oder eine geschützte Vorstufe hiervon eingesetzt werden.

Das Verhältnis der Bildung von anti (**1, 2**) bzw. syn (**2, 4**) -Verbindungen hängt von der Komponente der Strukturformel **6** ab.

In einer Ausführungsform wird die Reaktion in einem wässrigen Lösungsmittel durchgeführt. Vorzugsweise beträgt der Anteil an Wasser dann mehr als 50% und noch mehr bevorzugt mehr als 90%.

In einer anderen Ausführungsform wird die Reaktion ohne ein Lösungsmittel durchgeführt.

Besonders bevorzugte Reste R sind solche, in denen R -CH(OH)-CH₂-N₃ (*R* oder *S*) oder **6**

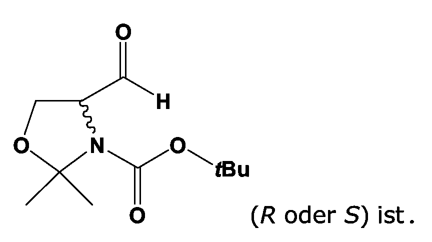

Gegenstand der Erfindung ist weiterhin die Verwendung der Verbindung **5** als Reagenz einer Aldolreaktion.

Ein weiterer Gegenstand der Erfindung sind Verbindungen der allgemeinen Formel 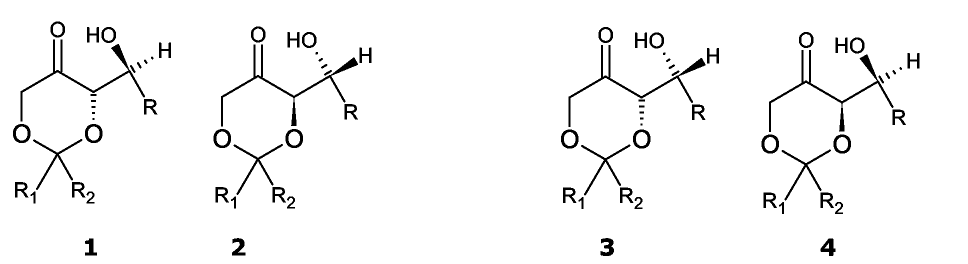 wobei
R = substituierte oder unsubstituierte Alkyl, Aryl, Heterozyklen enthaltend ein oder mehr O, N, S, P oder B;
R₁ und R₂ unabhängig voneinander H, substituierte oder unsubstituierte Alkyl, Aryl, Heterozyklen enthaltend ein oder mehr O, N, S, P oder B sind.

Die erfindungsgemäßen Substanzen, die auf dem neuen Syntheseweg in hohen Stereoselektivitäten erhalten werden können, eignen sich insbesondere als Ausgangs- bzw. Zwischenprodukte in der Synthese von Kohlenhydraten und Azakohlenhydraten. Daher ist auch die Verwendung der erfindungsgemäßen Verbindung als Zwischenprodukt in der Synthese von Kohlenhydraten oder Azakohlenhydraten Gegenstand der Erfindung.

Die Erfindung wird durch die nachfolgenden weiteren Beispielen näher erläutert.

### Beispiele

### Allgemeine Arbeitsvorschrift:

Eine Suspension von *L*-Prolin (20-30 Mol %), 5-Oxo-1,3-dioxane **5** (1.0 mmol), und ein Aldehyd **6** (1.0 mmol) in DMSO (0-8 mL) wurden bei Raumtemperatur für 12 bis 48 Stunden gerührt. Nach kompletter Umwandlung des Ausgangsmaterials (dünnschichtchromatographisch geprüft) wurde das Reaktionsgemisch durch Zugabe von gesättigter Ammoniumchloridlösung (3 mL), Extraktion mit Ethylacetat und Trocknung der organischen Phasen über Magnesiumsulfat aufgearbeit. Das Rohprodukt wurde durch Säulenchromatographie an Silicagel mit Heptan/Ethylacetat (3:1) weitergereinigt.

### NMR Daten

### (1'S, 4S)-4-(1'-Hydroxy)-propyl-2,2-dimethyl-[1,3]dioxan-5-on

Ausbeute: 56% 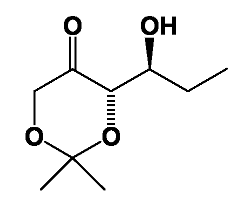 ^{**1**}**H NMR (250 MHz, CDCl**_{**3**}**):** δ = 0.97 (t, 3 H), 1.45, 1.49 (2s, 6 H), 1.51 - 1.81 (m, 2 H), 3.05 (br s, 1H, OH), 3.80 - 3.88 (m, 1 H), 3.98 - 4.13 (m, 2 H), 4.20 - 4.34 (m, 1 H);-
^{**13**}**C NMR (50 MHz, CDCl**_{**3**}**):** δ = 9.6, 23.8, 24.2, 25.6, 67.1, 72.1, 76.0, 101.3, 211.8.-

### (1'S, 4S)-4-(1'-Hydroxy-3'-methyl)-butyl-2,2-dimethyl-[1,3]dioxan-5-on

Ausbeute: 72 % 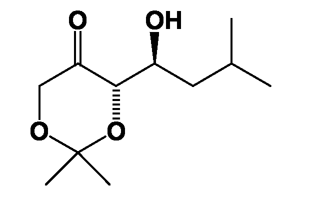 ^{**1**}**H NMR (250 MHz, CDCl**_{**3**}**):** δ = 0.85 - 0.98 (m, 8 H), 1.40 ( s, 3 H), 1.43 (s, 3 H), 1.72 - 1.94 (m, 1H), 2.99 (s, 1H, OH), 3.92 - 4.26 (m, 4 H); -
^{**13**}**C NMR (50 MHz, CDCl**_{**3**}**):** δ = 21.8, 23.8, 24.1 24.2, 41.5, 67.1, 69.6, 77.1, 101.2, 211.1.

### (1'S, 4S)-4-(1'-Hydroxy-1'-cyclohexyl)-methyl-2,2-dimethyl-[1,3]dioxan-5-on

Ausbeute: 70 % 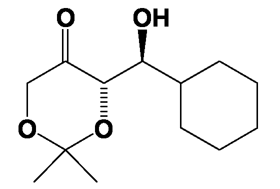 ^{**1**}**H NMR (250 MHz, CDCl**_{**3**}**):** δ = 1.01 - 1.33 (m, 5 H), 1.44, 1.48 (2s, 6 H), 1.55 - 1.89 (m, 6 H), 3.11 - 3.24 (br s, 1 H), 3.59 - 3.72 (m, 1 H), 3.93 - 4.04 (m, 1 H), 4.10 - 3.19 (m, 1 H), 4.19 - 4.32 (m, 2 H);-
^{**13**}**C NMR (50 MHz, CDCl**_{**3**}**):** δ = 24.0, 24.2, 26.5, 26.6, 26.8, 29.9, 38.8, 67.0, 73.8, 74.6, 101.3, 212.5.

### (1'S,4S)-4-(1'-Hydroxy-3'-phenyl)-propyl-2,2-dimethyl-[1,3]dioxan-5-on

Ausbeute: 80 % 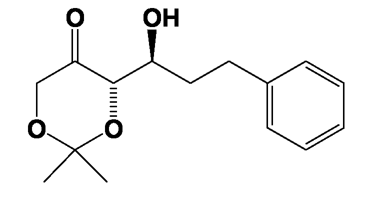 ^{**1**}**H NMR (250 MHz, CDCl**_{**3**}**):** δ = 1.48 (s, 3 H), 1.51 (s, 3 H), 1.78 - 2.11 (m, 2 H), 2.56 - 2.83 (m, 1H), 2.88 - 3.09 (m, 1 H), 3.18 (br s, 1 H, OH), 3.83 - 4.35 (m, 3 H), 7.13 - 7.45 (m, 5H);-
^{**13**}**C NMR (50 MHz, CDCl**_{**3**}**):** δ = 24.0, 24.2, 31.8, 34.6, 67.1, 70.3, 76.4, 101.5, 126.3, 128.8, 129.0, 142.4, 211.6.

### (1'S.4S)-4-(1'-Hydroxy-1'-phenyl)-methyl-2,2-dimethyl-[1,3]dioxan-5-on (anti)

### (1'R.4S)-4-(1'-Hydroxy-1'-phenyl)-methyl-2,2-dimethyl-[1,3]dioxan-5-on (syn)

Ausbeute: 77 % (gemeinsame Ausbeute beider Diastereomere)

In diesem Falle entstanden mit L-Prolin sowohl das anti- als auch das syn-Produkt. Sie konnten chromatographisch getrennt werden und zeigten die nachfolgenden NMR-Spektroskopiedaten. 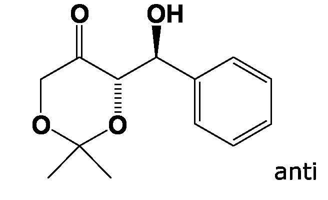 ^{**1**}**H NMR (250 MHz, CDCl**_{**3**}**):** δ = 1.29, 1.39 (2s, 6 H), 3.63 (br s, 1 H, OH), 3.92 - 4.39 (m, 3 H), 4.82 - 4.95 (m, 1 H), 7.34 - 7.51 (m, 5 H);-
^{**13**}**C NMR (50 MHz, CDCl**_{**3**}**):** δ = 23.6, 24.0, 67.1, 73.1, 76.6, 101.6, 127.5, 128.4, 139.7, 211.3. 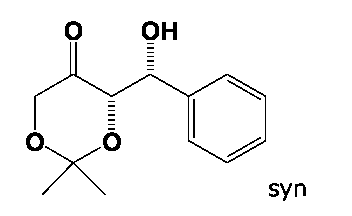 ^{**1**}**H NMR (250 MHz, CDCl**_{**3**}**):** δ = 1.38, 1.50 (2s, 6 H), 3.99 - 4.49 (m, 3 H), 5.02-5.20 (br s, 1 H, OH), 5.26 (d, 1 H, J = 2.7 Hz), 7.22 - 7.64 (m, 5 H);-
^{**13**}**C NMR (50 MHz, CDCl**_{**3**}**):** δ = 23.7, 24.7, 67.6, 71.7, 78.6, 101.4, 126.9, 128.7, 130.5, 140.7, 208.3.

## Patentansprüche

1. Verfahren zur Synthese von Verbindungen der allgemeinen Formel 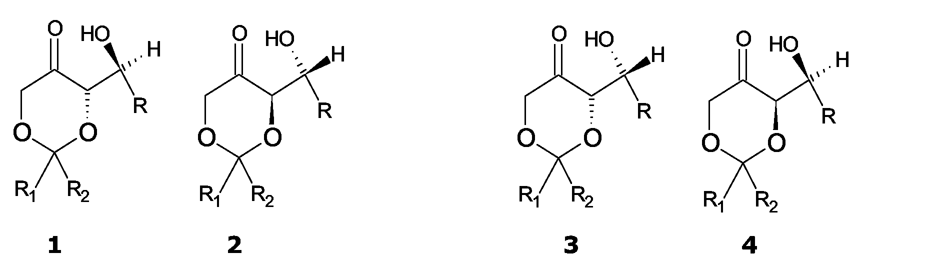 umfassend den Schritt der Umsetzung von 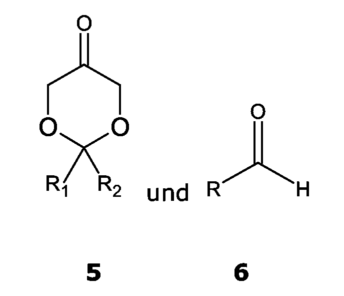 in Gegenwart von 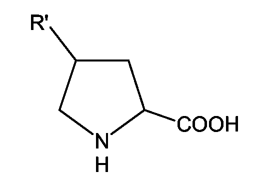 wobei
R = substituierte oder unsubstituierte Alkyl, Aryl, Heterozyklen enthaltend ein oder mehr O, N, S, P oder B;
R₁ und R₂ unabhängig voneinander H, substituierte oder unsubstituierte Alkyl, Aryl, Heterozyklen enthaltend ein oder mehr O, N, S, P oder B
R' = H, OH oder SiX₃ wobei X unabhängig voneinander Alkyl oder Aryl sind.

2. Verfahren nach Anspruch 1, **dadurch gekennzeichnet, dass** die Menge an Prolin 0.1 bis 30 Mol%, bezogen auf 1,3-Dioxan-5-onverbindung **5** beträgt.

3. Verfahren nach Anspruch 1 oder 2, **dadurch gekennzeichnet, dass** R ein oder mehr N enthält.

4. Verfahren nach mindestens einem der Ansprüche 1 bis 3, **dadurch gekennzeichnet, dass** R -CH(OH)-CH₂-N₃ (R oder S) ist oder **6**

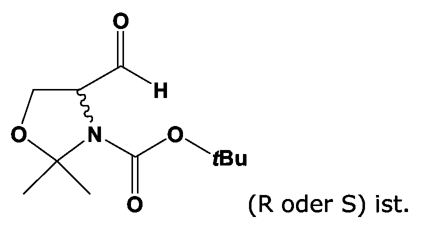

5. Verfahren nach mindestens einem der Ansprüche 1 bis 4, **dadurch gekennzeichnet, dass** die Reaktion in einem wässrigen Lösungsmittel durchgeführt wird.

6. Verfahren nach mindestens einem der Ansprüche 1 bis 6, **dadurch gekennzeichnet, dass** die Reaktion ohne Lösungsmittel durchgeführt wird.

7. Verfahren nach mindestens einem der Ansprüche 1 bis 6, **dadurch gekennzeichnet, dass** die Verbindungen **1, 3** durch Umsetzung mit L-Prolin und die Verbindungen **2, 4** durch Umsetzung mit D-Prolin erhalten wird.

8. Verwendung von 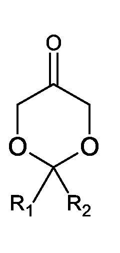 wobei
R₁ und R₂ unabhängig voneinander H, substituierte oder unsubstituierte Alkyl, Aryl, Heterozyklen enthaltend ein oder mehr O, N, S, P oder B
R' = H, OH oder SiX₃ wobei X unabhängig voneinander Alkyl oder Aryl sind als Reagenz in einer Aldolreaktion mit dem Katalysator

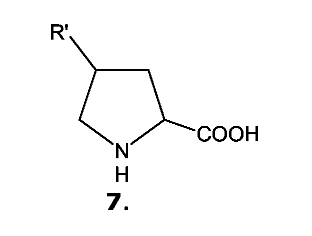

9. Verbindungen der allgemeinen Formel 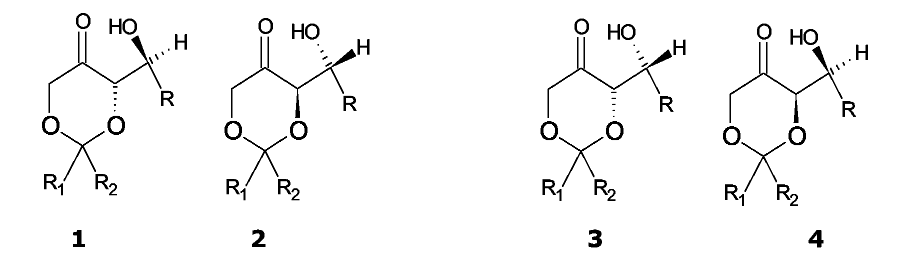 wobei
R = substituierte oder unsubstituierte Alkyl, Aryl, Heterozyklen enthaltend ein oder mehr O, N, S, P oder B;
R₁ und R₂ unabhängig voneinander H, substituierte oder unsubstituierte Alkyl, Aryl, Heterozyklen enthaltend ein oder mehr O, N, S, P oder B, ausgenommen Verbindungen mit R₁=R₂=CH₃.

10. Verwendung einer Verbindung nach Anspruch 7 als Zwischenprodukt in der Synthese von Kohlenhydraten und Aza-Kohlenhydraten.
